# EUROPEAN PATENT SPECIFICATION

(11) **EP 0 411 951 B1**
(45) Date of publication and mention of the grant of the patent: **30.11.1994**
(21) Application number: 90308551.2
(22) Date of filing: 03.08.1990
(51) Int. Cl.: C07C 409/16, C08B 37/16

(54) **Clathrate compounds of dicumyl peroxide with beta-cyclodextrins**
Einschlussverbindungen von Dicumylperoxid mit Beta-Cyclodextrinen
Composés d'inclusion du peroxide de dicumyle avec des béta-cyclodextrines

(30) Priority: 04.08.1989 JP 201219/89
(43) Date of publication of application: 06.02.1991
(73) Proprietor: A-ICS CORPORATION, Nagoya-shi, Aichi-ken (JP)
(72) Inventor: Osa, Tetsuo, Sendai-shi, Miyagi-ken (JP); Ueno, Akihiko, Sendai-shi, Miyagi-ken (JP); Osakada, Atsushi, Nagoya-shi, Aichi-ken (JP); Nakoji, Masateru, Okazaki-Shi, Aichi-ken (JP)
(74) Representative: Coleiro, Raymond

(56) References cited:
- JP-A-63 295 693
- CHEMICAL ABSTRACTS, vol. 99, no. 6, August 8, 1983, Columbus, Ohio, USA; H. TAGUCHI et al, "Free radical polymerization of water-soluble vinyl monomers in the two-phase (water/organic) system using methylated beta-cyclodextrins as initiator-carriers" page 4, abstract-no. 38 873u
- CHEMICAL ABSTRACTS, vol. 73, no. 15, October 12, 1970, Columbus, Ohio, USA; Y. MATSUI et al, "Stabilization of hydroperoxides by means of the formation of inclusion compounds with beta-cyclodextrin", page 314, right column, abstract no. 76 607f

## Description

The present invention relates to a clathrate compound comprising a specific organic peroxide as a guest compound and at least one natural or derived cyclodextrin as a host compound, as later described.

Organic peroxides such as dicumyl peroxide, tert-butylcumyl peroxide, benzoyl peroxide and the like have widely been used as crosslinking agents, free-radical reaction initiators, catalysts, curing agents, drying promotors, etc.

However, organic peroxides are chemically unstable and incapable of being safely kept for a long period of time, and may cause an explosion on receiving impact, resulting in both production and handling problems. The above organic peroxides are decomposed at such high speeds when at high temperatures that the temperature range within which they could be expected to be practically effective was limited.

On the other hand, it is known that cyclodextrin has a doughnut-like stereostructure, into a hollow space of which are taken in various guest compounds by clathration, or from the hollow space of which are taken out the various guest compounds.

Clathration between cyclodextrin as the host compound and known guest compounds generally provides various effects such as stabilization of volatile matter, antioxidation, deodorizing, relief from bitterness in bitter substances, emulsification of sparingly soluble substances, etc.

Further, uses of the known clathrate compounds comprising cyclodextrin as the host compound and known guest compounds cover various fields such as foods, medicines, agricultural chemicals, cosmetics, toiletries and plastics.

For example, M.L. Bender and M. Kimiyama, Cyclodextrin Chemistry, Springer-Verlag, Berlin, 1978, discloses clathrate compounds comprising cyclodextrin as the host compound and a guest compound such as diphenyl carbonate, diphenyl pyrophosphate or methylphosphonic acid diphenyl ester, and further discloses that the above guest compounds are very stable in a neutral and alkaline solution, but formation of clathrate compounds therefrom remarkably accelerates their hydrolysis.

JP-A-63-295693 discloses compositions for polymerization in which a polymerization initiator (which may be a peroxide, including as one of many, dicumyl peroxide) is present in a clathrate with an α; β or γ-cyclodextrin compound. The composition has improved pot life and the clathrate is capable of decomposition at a low temperature of 70 to 95°C to release the initiator for polymerization.

The present invention provides clathrate compounds, use of which results in making it possible to decompose an organic peroxide as the guest compound at higher temperatures and lower decomposition speed compared with the organic peroxide itself, and to improve certain properties of the organic peroxide as the guest compound, namely thermal stability, storage stability, photostability, impact resistance and solubility in water compared with the organic peroxide itself, resulting in making easy the handling of the organic peroxide used in the present invention and in widening the application of the organic peroxide used in the present invention.

That is, the present invention provides a clathrate compound consisting of dicumyl peroxide as a guest compound and, as a host compound, at least one cyclodextrin compound (which hereinafter may simply be referred to as cyclodextrin) selected from β-cyclodextrin, monomethylated-β-cyclodextrin, dimethylated-β-cyclodextrin, trimethylated-β-cyclodextrin and random-methylated β-cyclodextrin. The molar ratio of the guest compound:the host compound may be in the range of from 1:1 to 1:2 inclusive.

A natural cyclodextrin which may be used to obtain a clathrate compound embodying the present invention is prepared by the action of an enzyme on a starch, and is a doughnut-like non-reduced oligo-saccharide in which glucose is linked in a cyclic form through an α-1,4-linkage. The natural cyclodextrin containing 7 of the glucose moiety is employed because it has a hollow aperture of an inner diameter suitable for taking in the above organic peroxide as the guest compound by clathration. In particular, it has a hollow aperture of an inner diameter of 7.0Å and a hollow aperture depth of 7.0Å. It is found that this is suitable for taking in dicumyl peroxide.

Methyl derivatives of β-cyclodextrin which may be used to obtain a clathrate compound embodying the present invention may be those derived from natural β-cyclodextrin by subjecting it to a chemical reaction, for example, monomethylation, dimethylation, trimethylation, or random methylation (which hereinafter may be referred to as partial methylation).

Specific examples of the derived β-cyclodextrin include those obtained by subjecting one hydroxyl group at C-2, C-3, or C-6 of the glucose moiety of β-cyclodextrin to methylation (which products may be referred to hereinafter as monomethylated cyclodextrins); those obtained by subjecting respective hydroxyl groups at C-2 and C-3 of the glucose moiety of the β-cyclodextrin to methylation (which products may be referred to hereinafter as dimethylated cyclodextrins); those obtained by subjecting respective hydroxyl groups at C-2, C-3 and C-6 of the glucose moiety of the β-cyclodextrin to methylation (which products may be referred to hereinafter as trimethylated cyclodextrins); and those obtained by subjecting respective hydroxyl groups at C-2, C-3 and C-6 of the glucose moiety of the β-cyclodextrin to random methylation (which products may be referred to hereinafter as random-methylated cyclodextrins).

The derived cyclodextrin of the present invention may be prepared by reacting the natural cyclodextrin with barium oxide and dimethylsufuric acid or methyl iodide.

The natural cyclodextrin and the derived cyclodextrin in the present invention may be used alone or in combination.

The clathrate compound comprising the dicumyl peroxide as a guest compound and at least one of the natural and derived cyclodextrin as a host compound is such that the dicumyl peroxide as the guest compound is taken in by clathration into the central area of the hollow hole of the host compound having the doughnut-like stereostructure. The molar ratio of an amount of the dicumyl peroxide to that of the host compound is generally within the range of from 1:0.5 to 1:2.

The clathrate compound of the present invention may be prepared, for example, by adding the liquid or powdery organic peroxide to a heated aqueous host compound solution at 40 to 80°C, preferably 50 to 60°C, followed by thoroughly stirring for about 20 to 60 minutes, cooling down to room temperature, collecting precipitates thus formed by filtration, washing the resulting solids with water, washing with a small amount of an organic solvent which is capable of dissolving the free organic peroxide not subject to clathration, and drying under reduced pressure.

The clathrate compound of the present invention may also be prepared by directly thoroughly mixing the host compound and the organic peroxide. In this case, the organic peroxide is thoroughly mixed for about 10 to 30 minutes with the host compound at room temperature when the organic peroxide is liquid at room temperature, or at a temperature which is the melting point of the organic peroxide or higher and at which the organic peroxide is not decomposed, preferably at a temperature higher than the melting point by 5 to 10°C when the organic peroxide is solid at room temperature but is stable at and above the melting point of the organic peroxide to obtain a powder containing a clathrate compound. The resulting powder is washed with water, followed by washing with a small amount of an organic solvent which is capable of dissolving the free starting organic peroxide not subjected to clathration, and drying under reduced pressure to obtain a clathrate compound.

The latter process may be preferred from the industrial point of view.

Uses of the clathrate compound of the present invention may substantially be the same as those of the organic peroxide as the guest compound in the clathrate compound of the present invention except that the clathrate compound may be used under wider and more severe conditions compared with the organic peroxide, and may include its use as a crosslinking agent, free-radical reaction initiator, catalyst, curing agent or drying promotor.

According to the present invention, there is provided a clathrate compound, use of which results in making it possible to decompose the organic peroxide as the guest compound at higher temperatures and lower decomposition speed compared with the organic peroxide itself, and to improve certain properties of the organic peroxide as the guest compound, namely thermal stability, storage stability, photostability, impact resistance and solubility in water compared with the organic peroxide itself, resulting in making easy the handling of the organic peroxide used in the preset invention and in widening the application of the organic peroxide used in the present invention. In other words, the present invention provides clathrate compounds having highly improved properties enabling them to be easily handled and provides a wider range of uses compared with dicumyl peroxide alone. Thus, the use of the clathrate compound of the present invention allows certain reactions to start in water where this would have been impossible using the peroxide alone, makes for easy temperature control during reaction, increases the temperature range of certain reactions, and so forth.

The present invention is explained more in detail by the following Examples.

### Example 1

A beaker was charged with 1,00g of dicumyl peroxide (marketed by Nippon Oil & Fats Co., Ltd. under a trademark PERCUMYL-D, PURITY : 98% or higher) to be melted by heating at 50°C, and 4.20g of β-cyclodextrin was added to be thoroughly mixed for carrying out clathration. The clathrate compound-containing mixture was washed first with water and the with ethyl ether in a suitable amount respectively to wash away the free β-cyclodextrin and dicumyl peroxide which were not subjected to clathration. The resulting solid was subjected to elemental analysis. The results were as follows.

| | | |
|---|---|---|
| Determined: | C: 51.13%; | H: 6,70% |
| Calculated: | C: 51.28%; | H: 6.60% |
| (Chemical formula: C₆₀H₉₂O₃₇)⁽¹⁾ | | |

| | | |
|---|---|---|
| (1) Empirical formula of a clathrate compound having a molar ratio of 1:1 as dicumyl peroxide:β-cyclodextrin (see Example 1). | | |

The above results show that a clathrate compound comprising one mole of dicumyl peroxide and one mole of β-cyclodextrin is formed.

### Examples 2-7

Procedures of Example 1 were repeated except that β-cyclodextrin (Example 2), γ-cyclodextrin (Example 3, a comparative Example outside the invention), dimethyl-β-cyclodextrin (Examples 4 and 5) and partially methylated β-cyclodextrin (Examples 6 and 7) were used in the respective amounts shown in Table-1 with the results of elemental analysis as shown in Table-2, which show that clathrate compounds comprising one mole of guest compound and one mole of host compound were formed in Examples 1-5 respectively. The partially methylated β-cyclodextrin (PMCD) used in Examples 6 and 7 as shown in Table-1 is one marketed by SANRAKU Incorporated and having an average degree of methylation of 68-71%, a degree of methylation at C-2 of the glucose moiety of 58-62%, a degree of methylation at C-3 of the glucose moiety of 48-52% and a degree of methylation at C-6 of the glucose moiety of 98-100%.

### Example 8 (a comparative Example outside the invention)

The same dicumyl peroxide as in Example 1 is used for comparison without using the host compounds as in Examples 1-7.

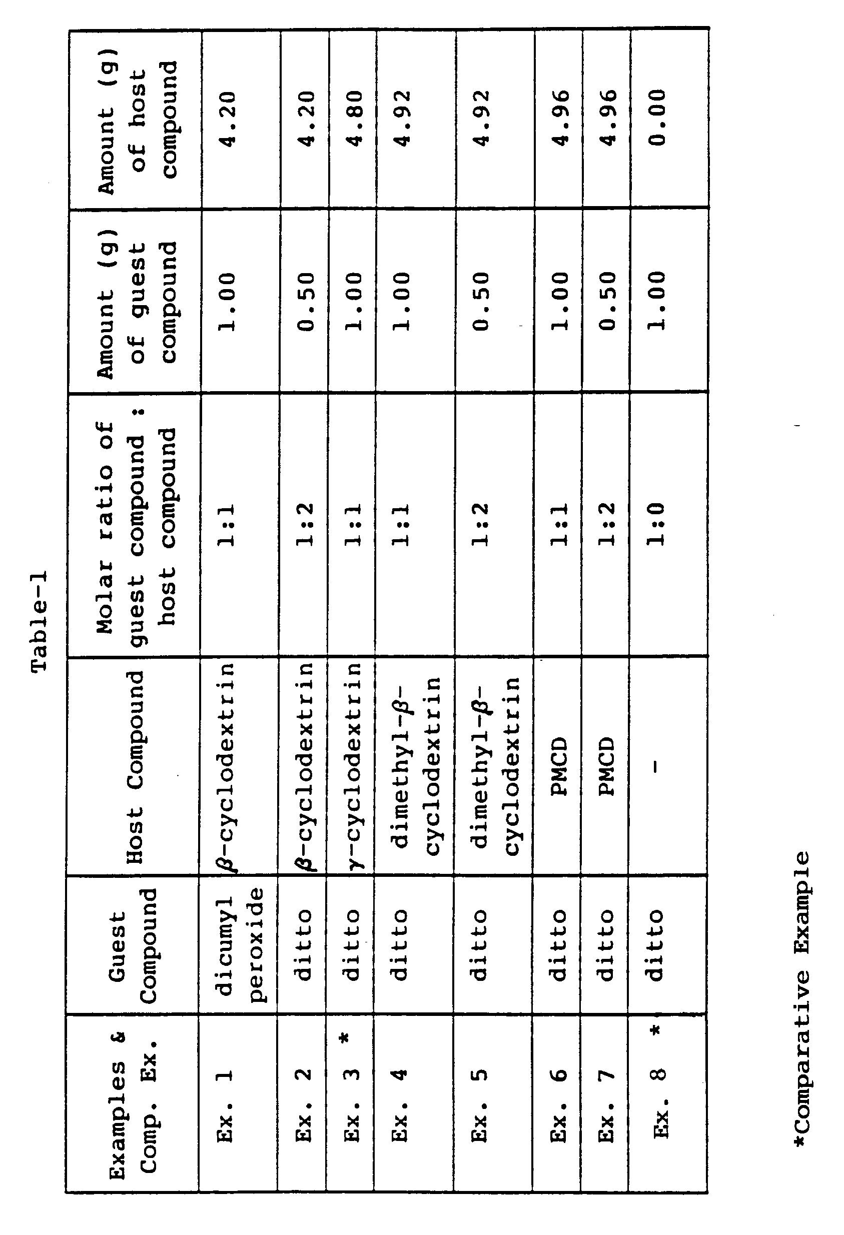

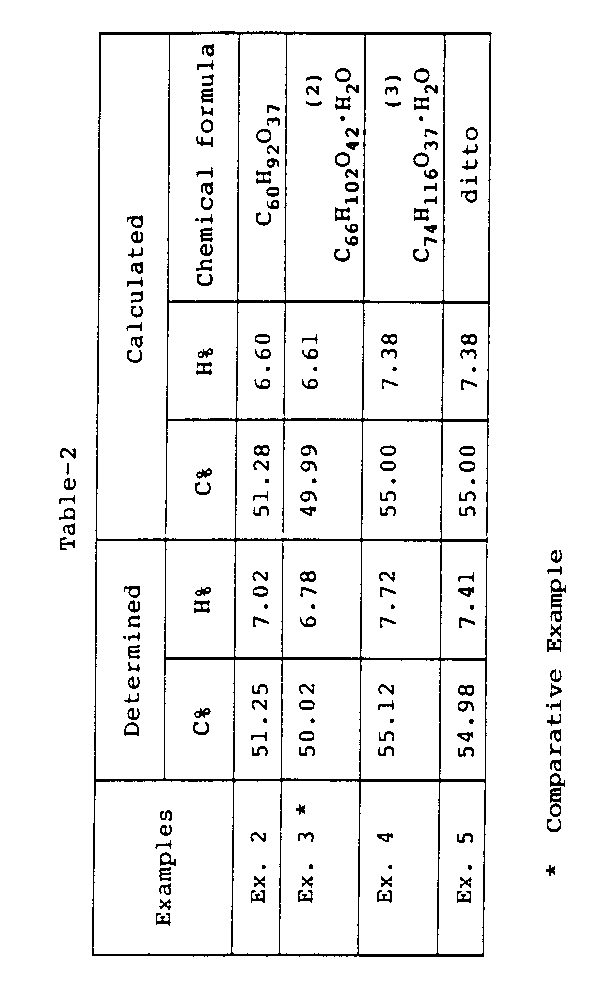

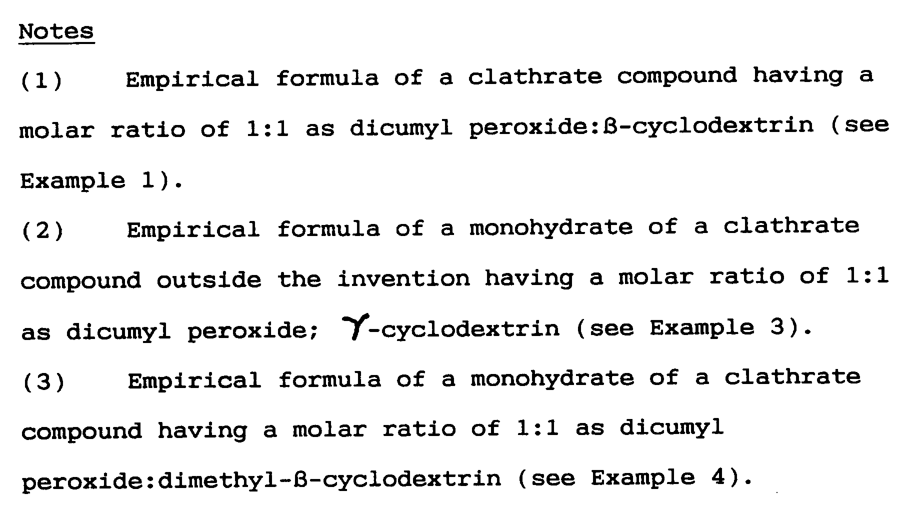

Thermal stability tests of the dicumyl peroxide as the guest compound compared with the dicumyl peroxide itself were carried out as follows.

Separately, a test tube equipped with a stopper and having a diameter of 15 mm and a length of 100 mm was charged with 0.15 g of respective clathrate compound-containing mixtures obtained after the completion of mixing for clathration in Examples 1-7, followed by sealing, leaving at rest in an oil bath (Riko MH-313 Type) at 125°C ± 0.5°C for a predetermined period of time as shown in Table-3, cooling with water, extracting with water and ethyl ether, separating the ethyl ether layer, distilling off the ethyl ether, and adding 0.5 ml of heavy chloroform for determination by nuclear magnetic resonance spectroscopy.

On the other hand, in Example 8, the same dicumyl peroxide as in Examples 1-7 was subjected to the same heating test as in Examples 1-7 except that the step of cooling with water was followed directly by adding 0.5 ml of heavy chloroform for dissolving without extracting with water and ethyl ether and by determining nuclear magnetic resonance (NMR) spectrum.

Thermal stability of the dicumyl peroxide as the guest compound and as the dicumyl peroxide itself was evaluated by determining the degree of thermal decomposition by the above nuclear magnetic resonance (NMR) spectroscopy as explained below.

Dicumyl peroxide decomposes by heating according to the following equation: 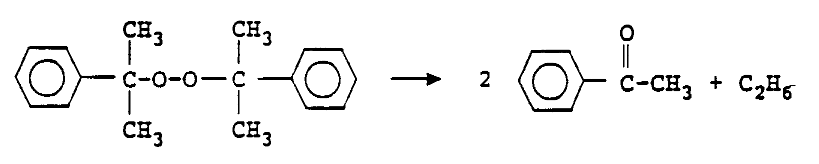
From the above equation, the following equation (I) is derived:

${\text{A}}_{\text{o}} {\text{= A}}_{\text{t}} {\text{+ 2B}}_{\text{t}} \text{(I)}$

where Aₒ is an initial value of an area of methyl proton in dicumyl peroxide on a NMR chart (at a time of t=0), Aₜ is an area of methyl proton in dicumyl peroxide on a NMR chart at a time of t, and Bₜ is an area of methyl proton in acetophenone on a NMR chart at a time of t.

The degree of thermal decomposition may be represented by the following equation (II): 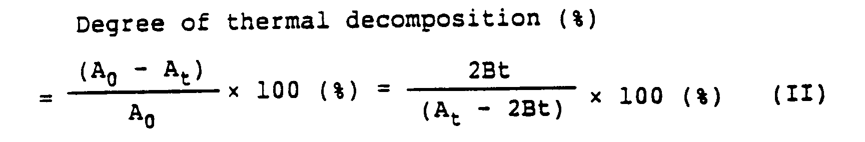
Changes of the degree of thermal decomposition with time during the above thermal stability tests are shown in Table-3.

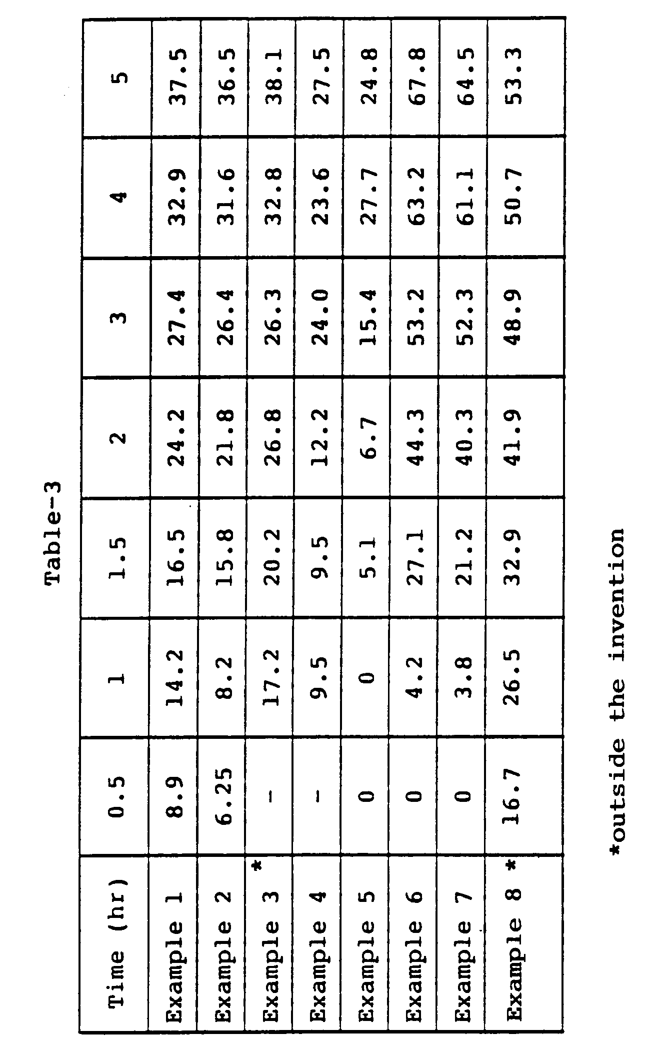

The results given in Table-3 show that the decomposition speed of the dicumyl peroxide as the guest compound of the clathrate compounds formed in Examples 1-5 (even including Example 3, outside the invention) is greatly reduced compared with that of the dicumyl peroxide itself in Example 8 (outside the invention). Thus, the thermal stability of the dicumyl peroxide as the guest compound of the clathrate compounds formed in Examples 1-5 is remarkably improved compared with that of the dicumyl peroxide itself in Example 8. Moreover, up to a period of 1 hour, the decomposition speed of the dicumyl peroxide in the clathrate of the β-cyclodextrin compounds of Examples 1, 2 and 4-7 is reduced as compared with that in the clathrate of the γ-cyclodextrin compound of Example 3. The thermal stability of the dicumyl peroxide as the guest compound of the clathrate compound is most improved in a clathrate compound comprising dicumyl peroxide as the guest compound and dimethyl-β-cyclodextrin as the host compound as shown in Example 5.

The results of Examples 6 and 7 show that the use of partially methylated β-cyclodextrin as the host compound seems to accelerate the decomposition speed of dicumyl peroxide as the host compound with time, but remarkably reduces the decomposition speed thereof for a certain period of time, for example, for about one hour from the beginning, resulting in showing remarkable thermal stability within a certain period of time, for example, about one hour under the conditions of Examples 6 and 7.

### Example 9

In 20ml of water at 60°C was dissolved 1g of β-cyclodextrin and 0.25g of dicumyl peroxide was added to the resulting solution to give a β-cyclodextrin:dicumyl peroxide molar ratio of 1:1, followed by stirring for 30 minutes so that the dicumyl peroxide is dispersed as oil drops. The resulting precipitates were collected on a glass filter to be washed with water, followed by washing with a small amount of ethyl ether under suction, and drying under reduced pressure to obtain a clathrate compound having a β-cyclodextrin: dicumyl peroxide molar ratio of 2:1.

The clathrate compound was subjected to elemental analysis with the following results:

| | | |
|---|---|---|
| Calculated: | C: 48.22; | H: 6.42% |
| (Chemical formula: C₁₀₂H₁₆₂O₇₂) | | |
| Determined: | C: 48.16%; | H: 7.03% |

### Example 10

One gram of dicumyl peroxide was heated at 50°C to melt it and 0.5g of β-cyclodextrin was added to give a β-cyclodextrin:dicumyl peroxide molar ratio of 1:8, followed by thoroughly mixing for clathration, washing the resulting product on a glass filter first with water and then with ethyl ether under suction, and drying under reduced pressure to obtain a clathrate compound having a β-cyclodextrin:dicumyl peroxide molar ratio of 1:1.

The clathrate compound was subjected to elemental analysis with the following results:

| | | |
|---|---|---|
| Calculated: | C: 51.28% | H: 6.60% |
| (Chemical formula: C₆₀H₉₂O₃₇) | | |
| Determined: | C: 51.57% | H: 7.06% |

## Claims

1. A clathrate compound consisting of dicumyl peroxide as a guest compound and, as a host compound, at least one cyclodextrin compound selected from β-cyclodextrin, monomethylated-β-cyclodextrin, dimethylated-β-cyclodextrin, trimethylated-β-cyclodextrin, and random-methylated-β-cyclodextrin.

2. A clathrate compound according to claim 1, wherein the cyclodextrin compound is dimethyl-β-cyclodextrin.

3. A clathrate compound according to claim 1, wherein the cyclodextrin compound is a random methylated-β-cyclodextrin methylated to an extent such that degrees of methylation at respective C-2, C-3 and C-6 positions of the glucose moieties are at least partial and an average degree of methylation of the glucose moieties is partial.

4. A clathrate compound according to claim 1, 2 or 3, wherein the molar ratio of the guest compound:the host compound being in the range from 1:1 to 1:2 inclusive.

5. A clathrate compound according to claim 4, wherein the molar ratio of the guest compound:the host compound is 1:2.

6. A clathrate compound according to claim 5, which consists of dicumyl peroxide and one said cyclodextrin compound in a molar ratio of 1:2.

7. Use of dicumyl peroxide as a crosslinking agent, characterised in that, at a temperature below the crosslinking, the dicumyl peroxide is present as a guest compound in a clathrate compound consisting of the dicumyl peroxide and, as a host compound, at least one cyclodextrin compound selected from β-cyclodextrin, monomethylated-β-cyclodextrin, dimethylated-β-cyclodextrin, trimethylated-β-cyclodextrin, and random-methylated-β-cyclodextrin.

## Patentansprüche

1. Clathratverbindung, bestehend aus Dicumylperoxid als Gastverbindung und aus zumindest einer Cyclodextrinverbindung als Wirtsverbindung, ausgewählt aus β-Cyclodextrin, monomethyliertem β-Cyclodextrin, dimethyliertem β-Cyclodextrin, trimethyliertem β-Cyclodextrin und zufalls- bzw. statistisch methyliertem β-Cyclodextrin.

2. Clathratverbindung nach Anspruch 1, worin die Cyclodextrinverbindung Dimethyl-β-cyclodextrin ist.

3. Clathratverbindung nach Anspruch 1, worin die Cyclodextrinverbindung zufalls- bzw. statistisch methyliertes β-Cyclodextrin ist, das in solchem Umfang methyliert ist, daß der Methylierungsgrad an der jeweiligen C-2, C-3 und C-6 Position der Glucoseeinheiten zumindest partiell und ein durchschnittlicher Methylierungsgrad der Glucoseeinheiten partiell ist.

4. Clathratverbindung nach Anspruch 1, 2 oder 3, worin das Molverhältnis von Gastverbindung : Wirtsverbindung im Bereich von 1 : 1 bis inklusive 1 : 2 liegt.

5. Clathratverbindung nach Anspruch 4, worin das Molverhältnis von Gastverbindung : Wirtsverbindung 1 : 2 beträgt.

6. Clathratverbindung nach Anspruch 5, die aus Dicumylperoxid und einer Cyclodextrinverbindung im Molverhältnis von 1 : 2 besteht.

7. Verwendung von Dicumylperoxid als Vernetzungsmittel, dadurch gekennzeichnet, daß unterhalb der Vernetzungstemperatur das Dicumylperoxid als Gastverbindung in einer Clathratverbindung vorliegt, die aus dem Dicumylperoxid und, als Wirtsverbindung, aus zumindest einer Cyclodextrinverbindung, ausgewählt aus β-Cyclodextrin, monomethyliertem β-Cyclodextrin, dimethyliertem β-Cyclodextrin, trimethyliertem β-Cyclodextrin und zufalls- bzw. statistisch methyliertem β-Cyclodextrin, besteht.

## Revendications

1. Composé clathrate consistant de peroxyde de dicumyle en tant que composé invité et,en tant que composé hôte; au moins un composé cyclodextrine choisi parmi la β-cyclodextrine, la monoéthylée-β-cyclodextrine, la diméthylée-β-cyclodextrine, la triméthylée-β-cyclodextrine, et la méthylée aléatoirement-β-cyclodextrine.

2. Composé clathrate selon la revendication 1 où le compose cyclodextrine est la diméthyl-β-cyclodextrine.

3. Composé clathrate selon la revendication 1, où le composé cyclodextrine est une méthylée aléatoirement-β-cyclodextrine méthylée à une étendue telle que les degrés de méthylation aux positions C-2, C-3 et C-6 respectives des moitiés glucose sont au moins partielles et qu'un degré moyen de méthylation des moitiés glucose et partiel.

4. Composé clathrate selon la revendication 1, 2 ou 3, dans lequel le rapport molaire du composé invité : composé hôte est dans l'intervalle de 1:1 à 1:2 inclus.

5. Composé clathrate selon la revendication 4, dans lequel le rapport molaire du composé invité : composé hôte est 1:2.

6. Composé clathrate selon la revendication 5, qui consiste de peroxyde de dicumyle et d'un dit composé ciclodextrine à un rapport molaire de 1:2.

7. Utilisation de peroxyde de dicumyle en tant qu'agent de réticulation, caractérisée en ce qu'à une température en dessous de la réticulation, le peroxyde de dicumyle est présent en tant que composé invité dans un composé clathrate consistant de peroxyde de dicumyle et, en tant que composé hôte, au moins un composé cyclodextrine choisi parmi β-cyclodextrine, la monométhylée-β-cyclodextrine, la diméthylée-β-cyclodextrine, la triméthylée-β-cyclodextrine, et la méthylée aléatoirement-β-cyclodextrine.
